# FASCICULE DE BREVET EUROPEEN

(11) **EP 1 138 677 B1**
(45) Date de publication et mention de la délivrance du brevet: **04.06.2003**
(21) Numéro de dépôt: 01400814.8
(22) Date de dépôt: 30.03.2001
(51) Int. Cl.: C07D 281/02, A61K 31/554

(54) **Nouveau procédé de préparation de la 11-amino-3-chloro-6,11-dihydro-5,5-dioxo-6-méthyl-dibenzo[c,f][1,2]-thiazépine et application à la synthèse de la tianeptine**
Verfahren zur Herstellung von 11-Amino-3-chlor-6,11-dihydro-5,5-dioxo-6-methyl-dibenzo[c,f][1,2]-thiazepin und Anwendung zur Synthese von Thianeptin
Process for the preparation of 11-amino-3-chloro-6,11-dihydro-5,5-dioxo-6-methyl-dibenzo[c,f][1,2]-thiazepine and application to the synthesis of tianeptine

(30) Priorité: 31.03.2000 FR 0004111
(43) Date de publication de la demande: 04.10.2001
(73) Titulaire: LES LABORATOIRES SERVIER, 92200 Neuilly sur Seine (FR)
(72) Inventeur: Blanchard, Jacky, 76190 Sainte-Marie-des-Champs (FR); Turbe, Hugues, 76360 Villers-Ecalles (FR); Brigot, Daniel, 76190 Sainte-Marie-des-Champs (FR)

(56) Documents cités:
- EP-A- 0 671 173
- CA-A- 995 213
- FR-A- 1 566 191

## Description

La présente invention concerne un nouveau procédé de synthèse industrielle de la 11-amino-3-chloro-6,11-dihydro-5,5-dioxo-6-méthyl-dibenzo[c,f][1,2]-thiazépine de formule (I): 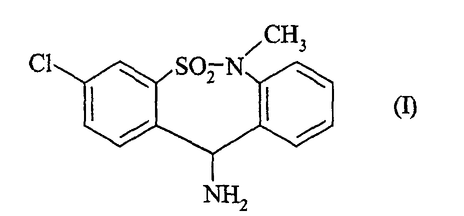 et de ses sels d'addition.

Le composé de formule (I) est un intermédiaire important dans la synthèse de la tianeptine de formule (II) : 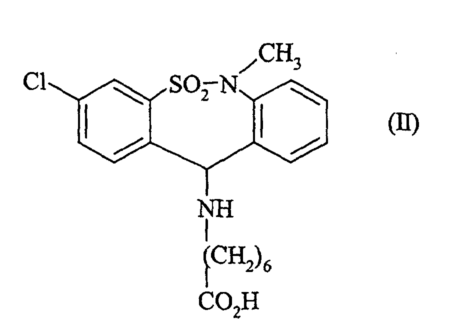 et de ses sels pharmaceutiquement acceptables.

Le composé de formule (II), ainsi que ses sels d'addition, possèdent des propriétés pharmacologiques intéressantes. Ce sont des stimulants de la capture de la sérotonine, ce qui les rend utiles dans le traitement de la dépression et de l'anxiété.

Le composé de formule (II), sa préparation et son utilisation en thérapeutique ont été décrits dans le brevet FR 2 104 728.

Compte-tenu de l'intérêt pharmaceutique de ce composé, il était important de pouvoir y accéder avec un rendement et une pureté optima, en partant de matières premières disponibles, peu onéreuses et en utilisant un procédé de synthèse facilement transposable à l'échelle industrielle.

Le brevet FR 2 104 728 décrit la préparation du composé de formule (II) par réaction de la 3,11-dichloro-6,11-dihydro-5,5-dioxo-6-méthyl-dibenzo[c,f][1,2]thiazépine avec le 7-amino-heptanoate d'éthyle.

Mais ce procédé ne permet pas d'obtenir le composé de formule (II) avec un rendement et une pureté satisfaisants, en raison notamment de l'instabilité du 7-aminoheptanoate d'éthyle dans le milieu réactionnel.

Les brevets CA 995 213 (page 3, lignes 11 à 15) et EP 0 671 173 décrivent respectivement la préparation du composé de formule (II) et la préparation d'un isomère du composé de formule (II) par réaction de l'amine de formule (I) avec le 7-bromo-heptanoate d'éthyle. Ce procédé est très intéressant, car il conduit à la tianeptine de formule (II) avec un rendement et une pureté bien meilleurs.
Mais l'accès à l'amine de départ de formule (I) n'est pas décrit avec précision.

En l'absence d'un procédé de synthèse de cet intermédiaire, des recherches approfondies ont été entreprises, qui ont abouti à la mise au point d'un procédé de synthèse industrielle particulièrement intéressant, permettant l'obtention en 2 étapes du composé de formule (I) avec un rendement et une pureté excellents, à partir de matières premières peu onéreuses.

L'invention a plus précisément pour objet un procédé de préparation du composé de formule (I) caractérisé en ce que l'on fait réagir la cétone de formule (III) : 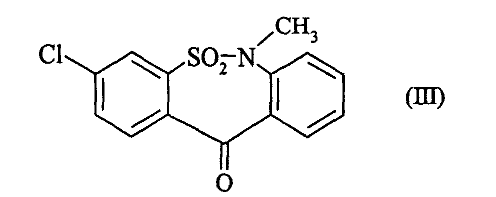 avec le borohydrure de sodium, en milieu biphasique consistant en un solvant chloré tel que, par exemple, le chloroforme, le dichlorométhane ou le dichloroéthane et une solution aqueuse d'hydroxyde de sodium, en présence de bromure de N-dodécyl-N-méthyl-diéthanolammonium, pour conduire à l'alcool de formule (IV): 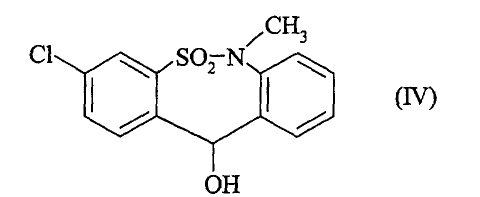 dont on traite la suspension dans un solvant chloré tel que, par exemple, le chloroforme ou le dichlorométhane, par l'acide chlorhydrique gazeux, pour conduire au chlorure de formule (V) : 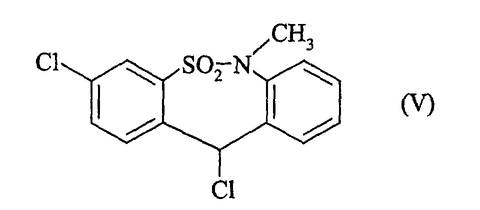 que l'on traite ensuite, sans l'isoler, par l'ammoniac gazeux en maintenant la température entre 25 et 35°C, pour conduire au composé de formule (I),
que l'on transforme si on le souhaite en un sel d'addition tel que le chlorhydrate.

Ce procédé est particulièrement intéressant pour les raisons suivantes :
- La réduction de la cétone de formule (III) par le borohydrure de sodium en milieu méthanolique est connue et a été notamment décrite dans le brevet FR 1 566 191. Cependant, le traitement de tels milieux réactionnels est laborieux à l'échelle industrielle, nécessitant en particulier l'évaporation de grandes quantités de méthanol. La demanderesse a trouvé qu'il était particulièrement avantageux d'effectuer cette réduction dans un solvant chloré tel que, par exemple, le dichloroéthane, le dichlorométhane ou le chloroforme, car l'alcool formé est alors directement isolable par filtration.
- La réduction des cétones par le borohydrure de sodium, lorsqu'elle est effectuée dans un solvant non hydroxylé, nécessite l'utilisation d'un catalyseur de transfert de phase. La demanderesse a trouvé que l'utilisation de bromure de N-dodécyl-N-méthyl-diéthanolammonium en quantité représentant 1 à 3 % en poids de la cétone engagée, permettait une réduction complète et très rapide (2 à 3 heures) de la cétone de formule (III). A titre de comparaison, l'utilisation d'hydrogénosulfate de tétrabutylammonium, catalyseur de transfert de phase courant, nécessite un temps de réaction deux fois plus long.
- L'alcool de formule (IV) ainsi obtenu est transformé en dérivé chloré de formule (V) par action d'acide chlorhydrique gazeux, puis le dérivé chloré, après dégazage, est traité in situ par l'ammoniac pour conduire à l'amine primaire de formule (I).
   Ce procédé présente l'avantage d'éviter l'isolement du dérivé chloré intermédiaire, limitant ainsi le nombre d'opérations.
- La préparation d'amines primaires par réaction de dérivés halogénés avec l'ammoniac donne généralement des résultats peu satisfaisants : les rendements sont faibles et il se forme des produits secondaires (amines secondaire et tertiaire) en proportion importante. La demanderesse a trouvé des conditions opératoires qui, de manière surprenante, permettent d'obtenir l'amine primaire de formule (I) avec de bons rendements à partir du chlorure correspondant de formule (V). Ces conditions consistent à faire passer un courant d'ammoniac à travers une suspension du chlorure de formule (V) dans un solvant chloré tel que le chloroforme ou le chlorure de méthylène, en maintenant la température du milieu réactionnel à environ 30°C. Le produit est ensuite avantageusement isolé du milieu réactionnel sous forme de chlorhydrate.
   Dans ces conditions, le rendement à partir de l'alcool de formule IV est supérieur à 75 % et le produit obtenu contient moins de 0,3 % d'amine secondaire de formule (VI) :

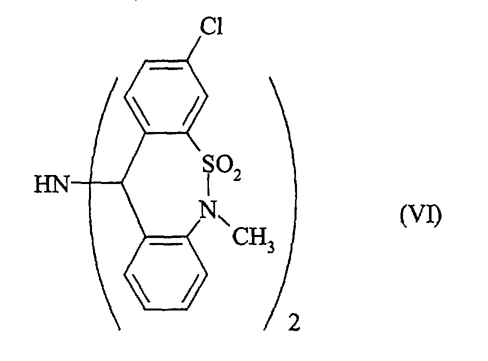

Le chlorhydrate ainsi obtenu a une très bonne pureté, ce qui rend son emploi particulièrement avantageux dans la synthèse de la tianeptine de formule (II).

A titre d'illustration, la réaction en milieu éthanolique à reflux du chlorhydrate de 11-amino-3-chloro-6,11-dihydro-5,5-dioxo-6-méthyl-dibenzo[c,f][1,2]thiazépine obtenu selon le procédé de l'invention avec le 7-bromoheptanoate d'éthyle, en présence d'hydrogénocarbonate de sodium, permet de conduire à la tianeptine de formule (II) avec une pureté et un rendement très satisfaisants.

Celle-ci est ensuite transformée, lorsqu'on le souhaite, en son sel de sodium, par ajout d'hydroxyde de sodium. Le sel de sodium de la tianeptine ainsi obtenu a une pureté excellente et contient moins de 0,4 % d'impuretés (mesurées par chromatographie liquide sur colonne C18).

Il contient en particulier moins de 0,1 % du produit de disubstitution de formule (VII) : 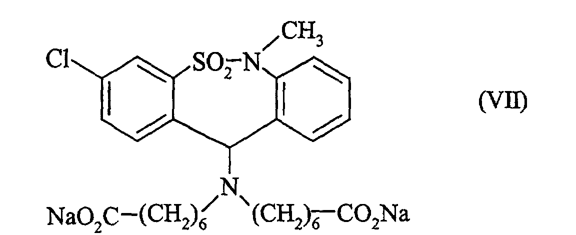 et ne contient pas l'impureté de formule (VI).

Les exemples suivants illustrent l'invention et ne la limitent en aucune façon.

### EXEMPLE 1: 3-Chloro-6,11-dihydro-5,5-doxo-11-hydroxy-6-méthyl-dibenzo [c,f][1,2]thiazépine

Dans un réacteur sous agitation sont introduits 100 kg de 3-chloro-6,11-dihydro-6-méthyl-5,5,11-trioxo-dibenzo[c,f][1,2]thiazépine, 1,8 kg de bromure de N-dodécyl-N-méthyl-diéthanolammonium et 100 l de chloroforme. Le mélange est ensuite porté au reflux, puis une solution de 4,6 kg de borohydrure de sodium dans 140 l d'eau et 0,7 kg de lessive de soude à 30 % est ajoutée. Après la fin du dégagement gazeux, le milieu réactionnel est ramené à température ambiante, le précipité obtenu est filtré, lavé à l'eau et séché. Le composé du titre est ainsi obtenu avec un rendement de 97 %.
Point de fusion : 199-200°C

### EXEMPLE 2: Chlorhydrate de 11-amino-3-chloro-6,11-dihydro-5,5-dioxo-6-méthyl-dibenzo[c,f][1,2]thiazépine

Une suspension de l'alcool décrit dans l'Exemple 1 (100 kg) dans le chloroforme est traitée à 5°C par un courant d'acide chlorhydrique gazeux, puis, après élimination de l'excès d'acide chlorhydrique par un dégazage à l'azote, la suspension de chlorure obtenue est traitée par un courant d'ammoniac gazeux en maintenant la température du milieu réactionnel à 30°C. L'excès d'ammoniac est ensuite éliminé par un courant d'azote, puis de l'eau est additionnée, le milieu est décanté, la phase organique est lavée à l'eau puis traitée par 30 kg d'acide chlorhydrique concentré. Le précipité obtenu est récolté par filtration, lavé par du chloroforme puis séché. On obtient ainsi le composé du titre avec un rendement de 79 %.
Point de fusion : 193-197°C

## Revendications

1. Procédé de synthèse industrielle de la 11-amino-3-chloro-6,11-dihydro-5,5-dioxo-6-méthyl-dibenzo[c,f][1,2]thiazépine de formule (I): 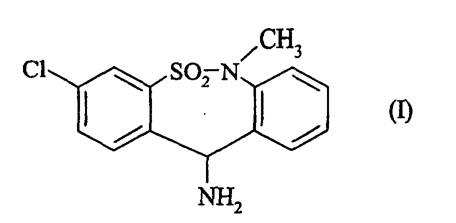 et de ses sels d'addition,
**caractérisé en ce que** l'on fait réagir la cétone de formule (III): 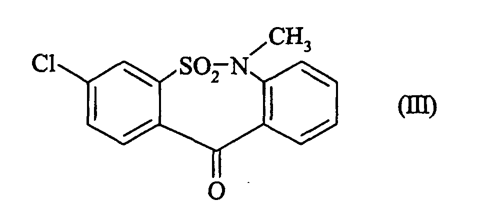 avec le borohydrure de sodium, en milieu biphasique consistant en un solvant chloré tel que, par exemple, le chloroforme, le dichlorométhane ou le dichloroéthane et une solution aqueuse d'hydroxyde de sodium, en présence de bromure de N-dodécyl-N-méthyl-diéthanolammonium, pour conduire à l'alcool de formule (IV) : 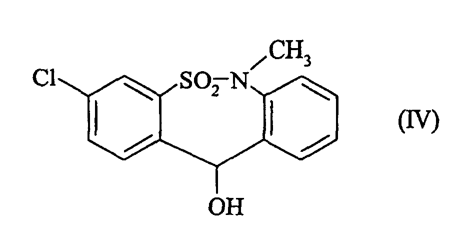 dont on traite la suspension dans un solvant chloré tel que, par exemple, le chloroforme ou le dichlorométhane, par l'acide chlorhydrique gazeux, pour conduire au chlorure de formule (V) : 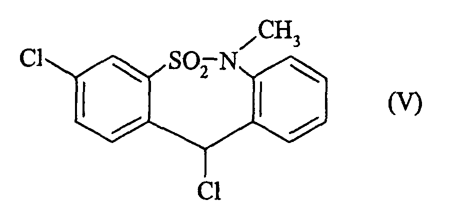 que l'on traite ensuite, sans l'isoler, par l'ammoniac gazeux en maintenant la température entre 25 et 35°C, pour conduire au composé de formule (I),
que l'on transforme si on le souhaite en un sel d'addition tel que le chlorhydrate.

2. Procédé selon la revendication 1 **caractérisé en ce que** le taux d'amine secondaire de formule (VI) dans le chlorhydrate du composé de formule (I) formé est inférieur à 0,3 %.

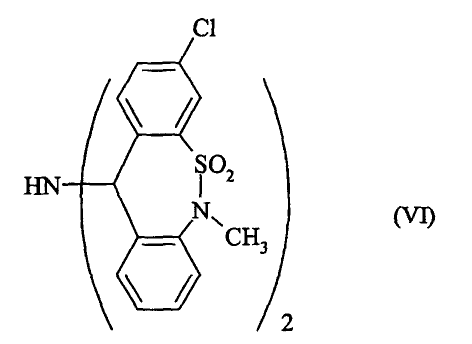

3. Procédé de synthèse de la tianeptine ou de ses sels pharmaceutiquement acceptables à partir de 11-amino-3-chloro-6,11-dihydro-5,5-dioxo-6-méthyl-dibenzo[c,f][1,2] thiazépine comme produit de départ, **caractérisé en ce que** ledit produit de départ est préparé selon le procédé de l'une quelconque des revendications 1 ou 2.

4. Procédé de synthèse du sel de sodium de la tianeptine à partir de 11-amino-3-chloro-6,11-dihydro-5,5-dioxo-6-méthyl-dibenzo[c,f][1,2] thiazépine comme produit de départ, **caractérisée en ce que** ledit produit de départ est préparé selon le procédé de l'une quelconque des revendications 1 ou 2.

5. Procédé selon la revendication 4 **caractérisé en ce que** le sel de sodium de la tianeptine obtenu a un taux d'impuretés inférieur à 0,4 %.

6. Procédé selon l'une quelconque des revendications 4 ou 5, **caractérisé en ce que** le sel de sodium de la tianeptine obtenu a un taux en impureté de formule (VII) inférieur à 0,1 %.

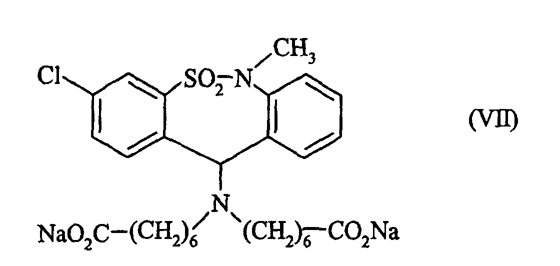

7. Procédé selon l'une quelconque des revendications 4 à 6, **caractérisé en ce que** le sel de sodium de la tianeptine obtenu ne contient pas l'impureté de formule (VI).

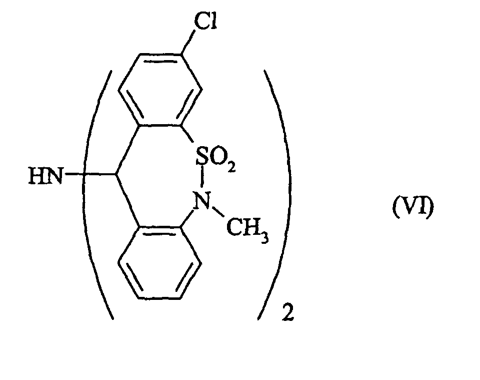

## Patentansprüche

1. Verfahren zur technischen Synthese von 11-Amino-3-chlor-6,11-dihydro-5,5-dioxo-6-methyl-dibenzo[c,f][1,2]thiazepin der Formel (I): 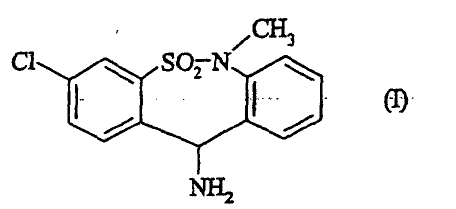 und seiner Additionssalze,
**dadurch gekennzeichnet, daß** man das Keton der Formel (III): 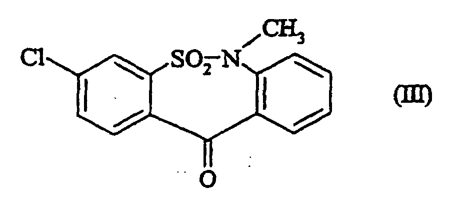 mit Natriumborhydrid in zweiphasigem Medium, welches aus einem Chlor-haltigen Lösungsmittel, wie beispielsweise Chloroform, Dichlormethan oder Dichlorethan, und einer wäßrigen Natriumhydroxidlösung besteht, in Gegenwart von N-Dodecyl-N-methyl-diethanolammoniumbromid umsetzt zur Bildung des Alkohols der Formel (IV): 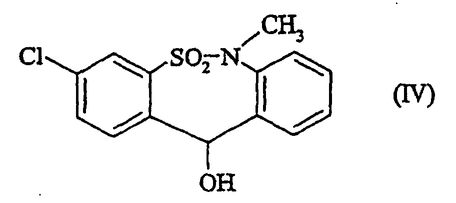 welchen man in der Suspension in einem Chlor-haltigen Lösungsmittel, wie beispielsweise Chloroform oder Dichlormethan, mit gasförmiger Chlorwasserstoffsäure behandelt zur Bildung des Chlorids der Formel (V): 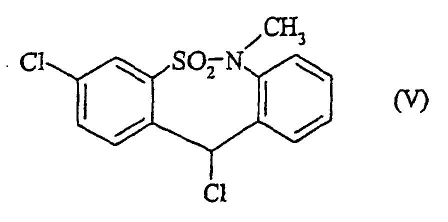 welches man anschließend ohne Isolierung mit gasförmigem Ammoniak behandelt unter Aufrechterhaltung einer Temperatur zwischen 25 und 35°C zur Bildung der Verbindung der Formel (I).
welche man gewünschtenfalls in ein Additionssalz, wie das Hydrochlorid, umwandelt.

2. Verfahren nach Anspruch 1, **dadurch gekennzeichnet, daß** der Gehalt an dem sekundären Amin der Formel (VI) 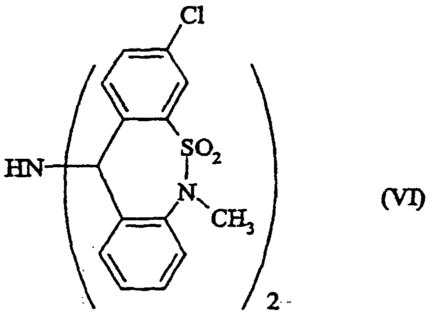 in dem Hydrochlorid der gebildeten Verbindung der Formel (VI) weniger als 0,3 % beträgt.

3. Verfahren zur Synthese von Tianeptin oder seiner pharmazeutisch annehmbaren Salze ausgehend von 11-Amino-3-chlor-6,11-dihydro-5,5-dioxo-6-methyl-dibenzo[c,f][1,2]thiazepin als Ausgangsprodukt,. **dadurch gekennzeichnet, daß** das Ausgangsprodukt nach dem Verfahren gemäß einem der Ansprüche 1 oder 2 hergestellt wird.

4. Verfahren zur Synthese von des Natriumsalzes von Tianeptin ausgehend von 11-Amino-3-chlor-6,11-dihydro-5,5-dioxo-6-methyl-dibenzo[c,f][1,2]thiazepin als Ausgangsprodukt, **dadurch gekennzeichnet, daß** das Ausgangsprodukt nach dem Verfahren gemäß einem der Ansprüche 1 oder 2 hergestellt wird.

5. Verfahren nach Anspruch 4, **dadurch gekennzeichnet, daß** das erhaltene Natriumsalz des Tianeptins einen Gehalt an Verunreinigungen von weniger als 0,4 % aufweist.

6. Verfahren nach irgendeinem der Ansprüche 4 oder 5, **dadurch gekennzeichnet, daß** das erhaltene Natriumsalz des Tianeptins einen Gehalt an der Verunreinigung der Formel (VII) 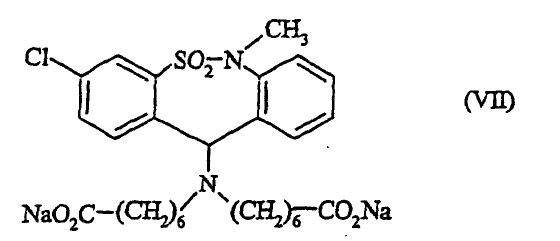 von weniger als 0,1 % aufweist.

7. Verfahren nach irgendeinem der Ansprüche 4 bis 6, **dadurch gekennzeichnet, daß** das erhaltene Natriumsalz des Tianeptins keine Verunreinigung der Formel (VI) 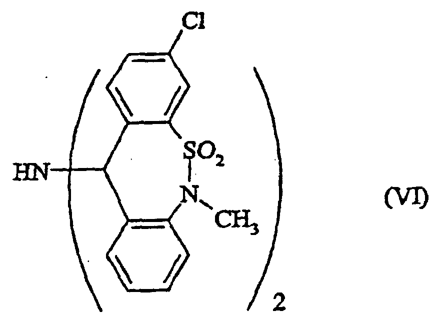 enthält.

## Claims

1. Process for the industrial synthesis of 11-amino-3-chloro-6,11-dihydro-5,5-dioxo-6-methyl-dibenzo[c,f][1,2]thiazepine of formula (I): 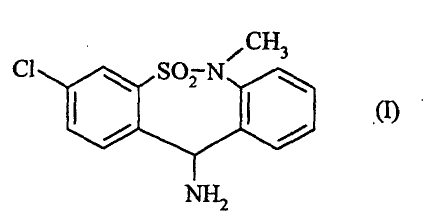 and its addition salts,
**characterised in that** the ketone of formula (III): 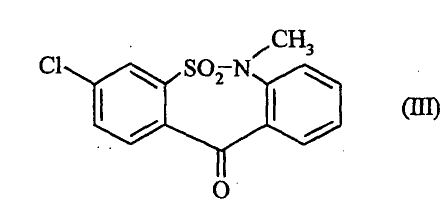 is reacted with sodium borohydride, in a two-phase medium consisting of a chlorinated solvent such as, for example, chloroform, dichloromethane or dichloroethane and aqueous sodium hydroxide solution, in the presence of N-dodecyl-N-methyl-diethanolammonium bromide, to yield the alcohol of formula (IV): 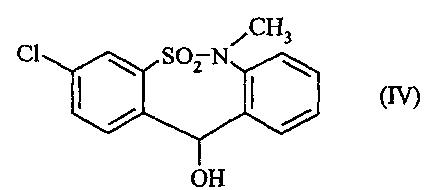 which, as a suspension in a chlorinated solvent such as, for example, chloroform or dichloromethane, is treated with gaseous hydrogen chloride to yield the chloride of formula (V): 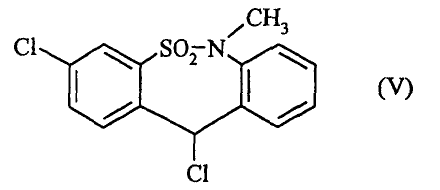 which is then, without being isolated, treated with gaseous ammonia, while maintaining the temperature between 25 and 35°C, to yield the compound of formula (I),
which is, if desired, converted into an addition salt such as the hydrochloride.

2. Process according to claim 1, **characterised in that** the level of the secondary amine of formula (VI) 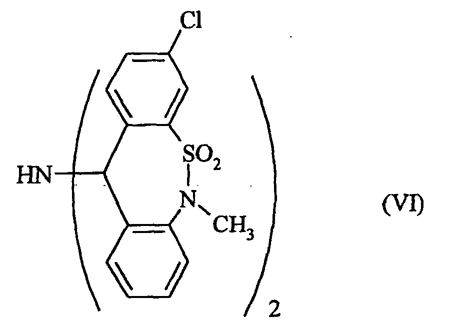 in the hydrochloride of the compound of formula (I) formed is less than 0.3 %.

3. Process for the synthesis of tianeptine or its pharmaceutically acceptable salts starting from 11-amino-3-chloro-6,11-dihydro-5,5-dioxo-6-methyl-dibenzo[c,f][1,2]thiazepine as starting compound, **characterised in that** the said starting compound is prepared according to the process according to either claim 1 or claim 2.

4. Process for the synthesis of the sodium salt of tianeptine starting from 11-amino-3-chloro-6,11-dihydro-5,5-dioxo-6-methyl-dibenzo[c,f][1,2]thiazepine as starting compound, **characterised in that** the said starting compound is prepared according to the process according to either claim 1 or claim 2.

5. Process according to claim 4, **characterised in that** the sodium salt of tianeptine obtained has a level of impurities of less than 0.4 %.

6. Process according to either claim 4 or claim 5, **characterised in that** the sodium salt of tianeptine obtained has a level of the impurity of formula (VII) 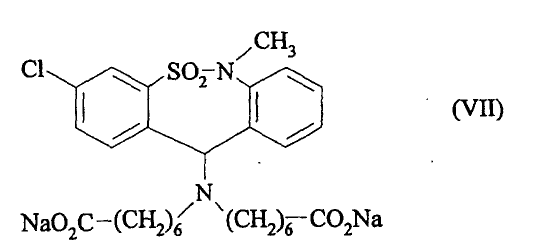 of less than 0.1 %.

7. Process according to any one of claims 4 to 6, **characterised in that** the sodium salt of tianeptine obtained does not contain the impurity of formula (VI)
